Europäisches Patentamt

European Patent Office

Office européen des brevets

(19)

(11) **EP 1 576 923 A1**

(12) **EUROPEAN PATENT APPLICATION**

(43) Date of publication:
21.09.2005 Bulletin 2005/38

(51) Int Cl.⁷: **A61B 5/053**

(21) Application number: 05005517.7

(22) Date of filing: 14.03.2005

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: 16.03.2004 JP 2004074528

(71) Applicant: Omron Healthcare Co., Ltd.
Kyoto 600-0084 (JP)

(72) Inventors:
• **Sato, Tetsuya**
**Ukyo-ku Kyoto-shi Kyoto 615-0084 (JP)**
• **Torii, Koji**
**Ukyo-ku Kyoto-shi Kyoto 615-0084 (JP)**

(74) Representative: **Kilian, Helmut**
**Wilhelms, Kilian & Partner**
**Patentanwälte**
**Eduard-Schmid-Strasse 2**
**81541 München (DE)**

(54) **Body composition measuring device with trend detection**

(57) In a display section, a measured value of information of a living body is displayed on a measured data display section (5A), and a figure representing a human body including body parts such as arm and leg is displayed on a body display section (5B). For example, a percentage of muscle and a percentage of body fat measured 30 days ago and a current percentage of muscle and percentage of body fat are compared with each other, and respective increase/decrease values are displayed on the measured data display section (5A) as well as the measured values. Further, a color of illumination in the background of the body display section (5B) is changed depending on a degree of the increase/decrease value. For example, the background of the body display section (5B) is illuminated in red when the percentage of muscle is decreased by a% or more, blue when increased by a% or more, and green when a percentage of variation is less than a% in comparing current data with data measured 30 days ago.

FIG.7A

**Description**

BACKGROUND OF THE INVENTION

Field of the Invention

The present invention relates to a body composition measuring device, in particular, a body composition measuring device for measuring a body composition including amount of body fat, percentage of body fat, lean body mass, percentage of lean body mass, area of visceral fat, amount of visceral fat, amount of muscle, percentage of muscle, moisture content and the like of a living body.

Description of the Background Art

In a known example of a body composition measuring device, a pair of electrodes is brought into contact with appropriate portions of a living body (for example, right hand and left hand, right foot and left foot, hand and leg, or the like), and a voltage is detected by applying a current to the pair of electrodes so that an impedance of the living body is measured, and further, a body weight is measured by a body weight scale or inputted, and an amount/percentage of body fat are measured based on the body weight and the measured impedance as well as age, gender and body height which are previously set and displayed.

In connection with the body composition measuring device of the foregoing type, a technology, wherein information, such as too thin, rather thin, sound, plump and obese, is displayed in a figure representing a shape of a human body using different colors depending on a measured amount of body fat, has been disclosed as a method of displaying the measured body fat (for example, see Japanese Laid-Open Patent Publication No. 07-204164).

As another example, a biometric measuring device, wherein an index relating to the body composition is displayed by means of a moving image in order to display a measurement result of the composition of the living body not only by using a numeral value but also in a visually comprehensible manner, has been disclosed (for example, see Japanese Laid-Open Patent Publication No. 2004-8659). As a specific method of the animated display, for example, a body part is displayed on a section of the figure having the human-body shape and a body composition value is displayed on a graphic to be thereby instantly comprehended.

Further, a healthcare guideline advising device, wherein an obesity index is synthetically determined based on the combination of a calculated percentage of body fat and an index of physical constitution and an index figure having the shape of the human body and corresponding to the obesity index is displayed on a display area apart from a digital display area of the measured value, has been disclosed (for example, see Japanese Laid-Open Patent Publication No. 2002-191573).

Further, a control-related technology, wherein healthcare index data such as body weight, percentage of body fat and blood pressure is compared with comparison data which is previously set, and a display color and a background display color of the healthcare index data displayed on a display section are changed depending on a result of the comparison, has been disclosed (for example, see Japanese Laid-Open Patent Publication No. 2002-159450).

When the amount/percentage of body fat are measured in the body composition measuring device, it is desirable to know if a favorable progress can be seen in a current measurement result in comparison with any past measurement result rather than merely knowing a measured value. In order to respond to the request, a technology, wherein the measurement result is compared with a standard value and a reference value which are previously set, and figures having shapes of obese, sound and thin human bodies are selectively displayed and further preferably displayed in different colors depending on a result of the comparison so that the measurement result can be instantly evaluated, has been proposed for the conventional body composition measuring devices.

The body composition measuring device is generally used because, for example, a user wishes to know the evaluation of the amount/percentage of body fat relative to the reference value or the user wishes to know the evaluation of a result obtained by his/her continuous physical exercise or dietary restriction. It would be convenient to be able to know if the current measured value is improved in comparison with the past value in the performed measurement.

However, in most of the body composition measuring devices recited in the foregoing publication, the displayed comparison result merely shows the result of the comparison relative to the standard value and the reference value, and it is not possible to know a level of the user's achievement in decreasing his/her amount of body fat as a result of the exercise or the like.

SUMMARY OF THE INVENTION

The present invention was implemented based on the foregoing disadvantage, and a main object thereof is to provide a body composition measuring device wherein the user can easily confirm if his/her body composition has been improved since when he/she was subjected to the measurement in the past.

In a body composition measuring device according to the present invention, information of a living body such as percentage of body fat and percentage of muscle is measured. The body composition measuring device includes a measuring section, a memory, a measured data comparing section, a measured data display section, a body display section and a body display controller. The measuring section measures the information of the living body in an entire body or respective parts of the body. The memory stores measured data of the living body information. The measured data comparing section compares the measured data of the living body information stored in the memory with updated measured data of the living body information. The measured data display section displays the measured data of the living body information. The body display section displays information including an image representing a shape of a human body including respective body parts such as arm and leg. The body display controller controls a display mode of the body display section depending on a result of the comparison of the measured data by the measured data comparing section.

According to the present invention, the current measured data is compared with the data measured in the past and stored, and the display mode in the body display section is changed depending on the comparison result. In the foregoing manner, a subject of measurement can immediately and intuitively know how much the living body information has changed and how much effect has been gained from the exercise, dietary restriction and the like he/she was committed to since when he/she was subjected to the measurement in the past, and further, can be easily aware of any change made in his/her entire body or respective body parts and a trend of the change.

The foregoing and other objects, features, aspects and advantages of the present invention will become more apparent from the following detailed description of the present invention when taken in conjunction with the accompanying drawings.

BRIEF DESCRIPTION OF THE DRAWINGS

Fig. 1 is an outline view of a body composition measuring device according to an embodiment of the present invention;
Fig. 2 shows a display section and a manipulating section of a main body of the body composition measuring device according to the embodiment;
Fig. 3 is a block diagram illustrating a circuit constitution of the body composition measuring device according to the embodiment;
Fig. 4 shows an example of data contents stored in an external memory of the body composition measuring device according to the embodiment;
Figs. 5A, 5B, 6A, 6B, 7A, 7B and 8 respectively show examples of displayed values in the body composition measuring device according to the embodiment;
Figs. 9 and 10 are flowcharts used for describing a measuring operation of the body composition measuring device according to the embodiment; and
Fig. 11 is a flowchart used for describing a display operation performed when a current value is evaluated relative to a past value in the body composition measuring device according to the embodiment.

DESCRIPTION OF THE PREFERRED EMBODIMENTS

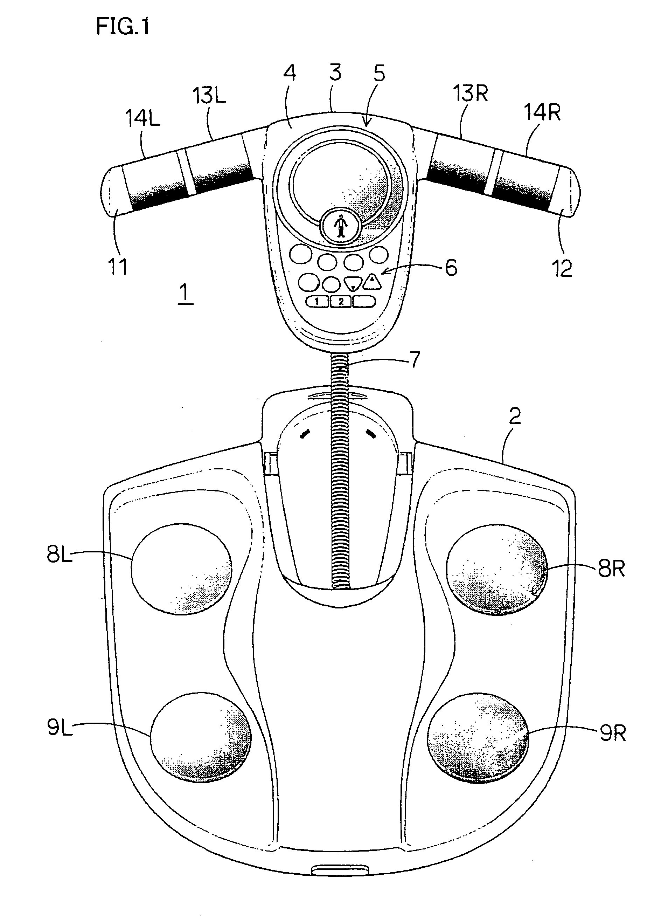

Hereafter, the present invention is described in detail referring to a preferred embodiment of the present invention. Referring to Fig. 1, a body composition measuring device 1 according to the embodiment includes a body weight scale 2 and a body composition measuring device main body 3. Body weight scale 2 and body composition measuring device main body 3 are electrically connected to each other by means of an elastic code 7.

Body weight scale 2 includes a distortion sensor for measuring a body weight inside thereof and, also, has left-foot electrodes 8L and 9L and right-foot electrodes 8R and 9R for measuring an impedance of a living body on a surface thereof Left-foot electrode 8L and right-foot electrode 8R serve to apply a high-frequency current and are electrically connected to each other inside a case which covers an outer frame of body weight scale 2. Left-foot electrode 9L and right-foot electrode 9R are measuring electrodes and also electrically connected to each other inside the case.

Body composition measuring device main body 3 includes a main body unit 4, a left grip section 11 and a right grip section 12. Main body unit 4 includes a display section 5 and a manipulating section 6. Left grip section 11 and right grip section 12 are integrally provided on both sides of main body unit 4, which form a shape of handlebars of a bicycle. Left-hand electrodes 13L and 14L are respectively provided in left grip section 11, while right-hand electrodes 13R and 14R are respectively provided in right grip section 12. Electrodes 13L, 14L, 13R and 14R are connected to a circuit section in main body unit 4 inside the case.

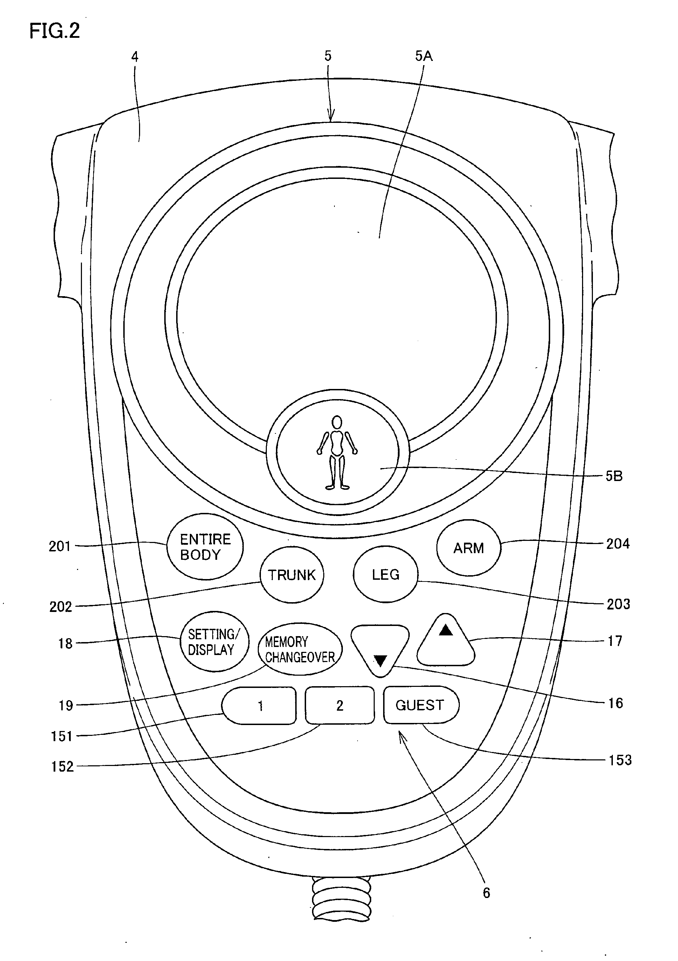

Referring to Fig. 2, display section 5 has a numeral value display part 5A having a large circular shape for showing a measured value and the like using a digital numeral value, and a determination display part 5B having a small circular shape for showing a determination result. An icon representing a shape of a human body (hereinafter, referred to as "human-shape icon") is displayed on determination display part 5B. Manipulating section 6 includes keys 151 and 152 for identifying a subject of measurement, a guest key 153, a DOWN key 16, an UP key 17, a setting/display changeover key 18, a memory changeover key 19, and further, an entire body key 201, a trunk key 202, a leg key 203 and an arm key 204 for selecting and setting a determination mode.

Keys 151 and 152 are the keys for designating and inputting "individual 1" and "individual 2" which are previously registered. Key 153 is used when a person other than the registered individuals, that is, a guest uses the measuring device of the present invention. DOWN key 16 and UP key 17 are manipulated when a marker or a numeral value is increased/decreased. Setting/display changeover key 18 is used when an item is set and a display changeover is carried out. Memory changeover key 19 is manipulated when point (past) data stored in a memory is read. Entire body key 201 is the key for designating an entire body measurement mode, while trunk key 202, leg key 203 and arm key 204 are used for respectively designating measurement modes of the relevant parts, which are a trunk measurement mode, a leg measurement and an arm measurement mode.

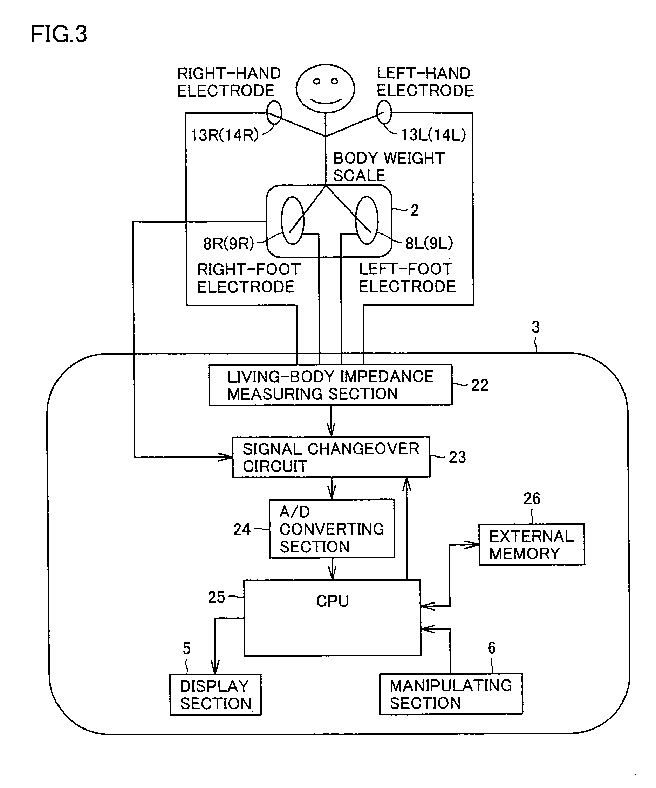

As described earlier, body weight scale 2 of body composition measuring device 1 incorporates the distortion sensor for measuring the body weight and includes right-foot electrode 8R (9R) and left-foot electrode 8L (9L) for measuring the impedance of the living body as shown in Fig. 3. Right-foot electrode 8R and left-foot electrode 8L are the electrodes for applying the high-frequency current, while right-foot electrode 9R and left-foot electrode 9L are the measurement electrodes. Body composition measuring device main body 3 includes display section 5, manipulating section 6, a living-body impedance measuring section 22, a signal changeover circuit 23, an A/D (Analog/Digital) converting section 24, a CPU (Central Processing Unit) 25 and an external memory 26.

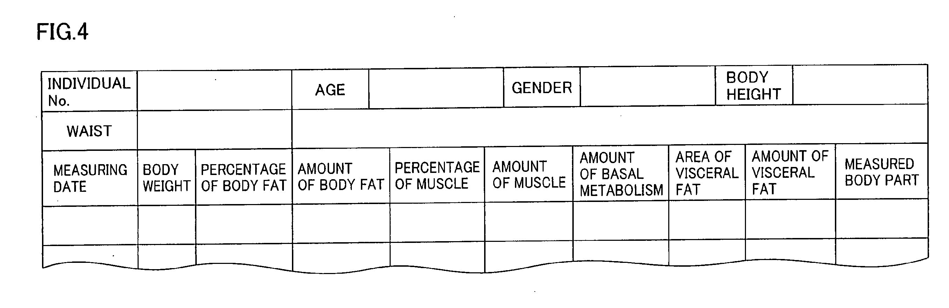

Living-body impedance measuring section 22 is connected to right-foot electrode 8R (9R), left-foot electrode 8L (9L), right-hand electrode 13R (14R) and left-hand electrode 13L (14L) so as to detect a voltage between the measurement electrodes and measure the living-body impedance. Living-body impedance measuring section 22 is capable of measuring the impedances in the respective body parts such as the entire body and trunk, and the leg or the arm. Signal changeover circuit 23 executes a changeover between a measured body weight signal outputted from body weight scale 2 and the living-body impedance measured by living-body impedance measuring section 22 in response to a changeover instruction from CPU 25 to thereby output one of them. A/D converting section 24 coverts an analog signal outputted from signal changeover circuit 23 into a digital signal and inputs the digital signal to CPU 25. CPU 25 has a function of calculating an amount of body fat, a percentage of body fat, an amount of lean body mass, a percentage of lean body mass, an area of visceral fat, an amount of visceral fat, a percentage of muscle and other body composition data for the entire body and the respective body parts based on gender, age and body height previously inputted from manipulating section 6 and registered, body weight outputted from body weight scale 2 and living-body impedance inputted from living-body impedance measuring section 22. External memory 26 stores the body composition data which was measured in the past. As shown in Fig. 4, data by each individual and each body part, for example, is stored in external memory 26. When memory changeover key 19 of manipulating section 6 is manipulated, the data measured seven days ago, 30 days ago and 90 days ago can be called up.

The present embodiment has a feature in that the measured body composition is compared with the past data so that a rate of any variation generated therebetween is determined and displayed in different colors depending on the rate of the variation, and further, the comparison of the body composition to the past data can be determined based on the entire body mode or the body part modes.

Referring to the determination of the rate of the variation, below is described the determination of the rate of the variation in comparison with the past data.

In the determination process, first, a percentage of variation P is obtained by means of the percentage of muscle and the percentage of body fat as shown in the following formula.

$$P = f \text{ (percentage of muscle, percentage of body fat) (\%)}$$

To be more specific, the percentage of variation P is obtained as follows.

Providing that the percentage of muscle and the percentage of body fat on a particular day when the comparison is carried out are respectively A% and B%, while the percentage of muscle and the percentage of body fat are currently C% and D%, the percentage of variation P is calculated by means of the following formula.

$$P = [\{(C/D) - (A/B)\}/(A/B)] \times 100 \ (\%)$$

Below is further described a case in which the comparison is carried out using the data measured seven days ago as the past data as a specific example of the percentage of variation P.

Providing that the percentage of muscle and the percentage of body fat measured seven days ago are respectively 28.0% and 30.0%, while the current percentage of muscle is 25.0% and the current percentage of body fat is 32.0%, the percentage of variation P is calculated as "-16.13%" based on the following formula.

$$P = [\{0.78 - 0.93\}/(0.93)] \times 100 \ (\%)$$

When the percentage of variation P is equal to or more than ±a%, or less than ±a%, a color determination is carried out. The color determination is carried out with respect to the entire body mode and each body part mode (corresponding to entire body key 201, trunk key 202, leg key 203 and arm key 204).

When the entire body mode is selected and the percentage of variation is equal to or less than -a% (percentage of muscle is decreased), a background portion of determination display part 5B is illuminated in red, a block corresponding to a part of the respective body parts in which the percentage of variation is equal to or less than -a% is blinked, and blocks corresponding to the rest of the body parts (in which the percentage of variation does not fall under the range of equal to or less than -a%) are illuminated. When the percentage of variation is less than ±a% (variation of less than ±a%), determination display part 5B is illuminated in green, while the blocks corresponding to the respective body parts are not blinked. Next, when the percentage of variation is equal to or more than +a% (muscle is increased), the background portion of determination display part 5B is illuminated in blue, a block corresponding to a part of the respective body parts in which the percentage of variation is equal to or more than +a% is blinked, and blocks corresponding to the rest of the body parts (in which the percentage of variation does not fall under the range of equal to or more than +a%).

As described above, in the entire body mode, it is learnt how the percentage of muscle is changed from the color of determination display part 5B when the entire body is observed. Further, it is learnt in which body part the percentage of muscle has remarkably varied from observing which block is blinked.

Next, when the respective body part modes are selected and the percentage of variation is equal to or less than -a% (percentage of muscle is decreased), the background portion of determination display part 5B is illuminated in red, and the relevant body part is also illuminated. When the percentage of variation is less than ±a% (variation of less than ±a%), the background portion of determination display part 5B is illuminated in green, and the selected body part is also illuminated. Herein, "a" recited in the foregoing description is a predetermined value. Next, when the percentage of variation is +a% (percentage of muscle is increased), the background portion of determination display part 5B is illuminated in blue, and the relevant part is also illuminated.

In the respective body part modes, it is learnt in which body part and how the percentage of muscle is changed from observing the color of determination display part 5B and identifying the illuminated block.

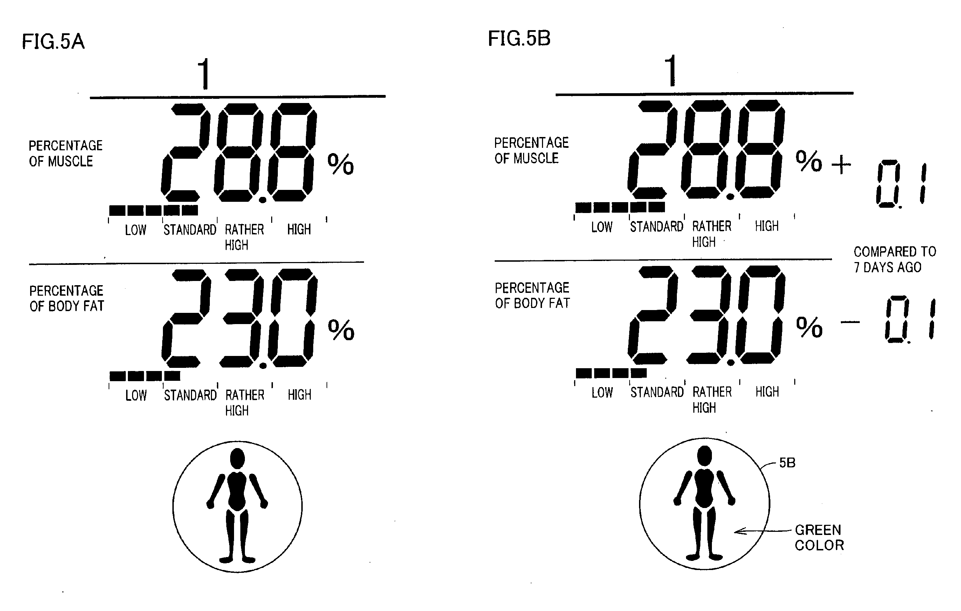

Next, specific examples of the display in the respective modes are described. A screen displaying a measurement result in the entire body mode is shown in Fig. 5A, in which the percentage of muscle is 28.8% and the percentage of body fat is 23.0%.

In the state in which the numeral values are displayed as in Fig. 5A, memory changeover key 19 is appropriately manipulated. Then, CPU 25 reads out the data of seven days ago to thereby calculate a variation width, and displays a relevant percentage of the variation on display section 5 as well as the measurement result shown in Fig. 5A, as shown in Fig. 5B. Fig. 5B exemplifies a case in which a differential value in the percentage of muscle is +0.1 % and a differential value in the percentage of body fat is -0.1 %. In the example shown in Fig. 5B, the percentage of variation calculated by means of the percentages of muscle and body fat exceeds -a% and falls less than a%. Therefore, the background portion of determination display part 5B is illuminated in green, and the human-shape icon is not blinked but ceaselessly illuminated.

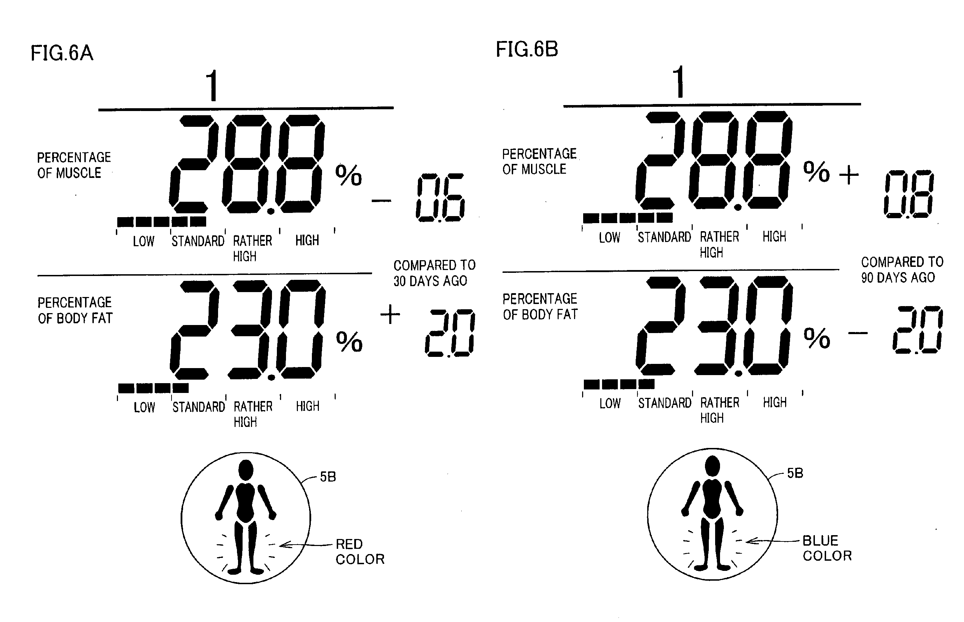

Subsequent to the foregoing process, when memory changeover key 19 is manipulated, the CPU 25 reads out the data of 30 days ago. CPU 25, as shown in Fig. 6A, displays the measurement result shown in Fig. 5A and information relating to the change of the percentages of muscle and body fat between the relevant measurement result and the data of 30 days ago on the display section 5. Fig. 6A exemplifies a case in which the differential value in the percentage of muscle is -0.6% and the differential value in the percentage of body fat is +2.0%. In the example shown in Fig. 6A, the percentage of variation is equal to or less than -a%. Accordingly, the background portion of determination display part 5B is illuminated in red, and further, only a leg part of the human-shape icon is displayed in the blinking manner because the percentage of muscle in the leg part has remarkably decreased.

Then, memory changeover key 19 is manipulated, CPU 25 reads out the data of 90 days ago. CPU 25 calculates the change of the percentages of muscle and body fat relative to the current measurement result (differential value) and displays the calculation result as well as the measurement result shown in Fig. 5A on the display section 5 as shown in Fig. 6B. Fig. 6B exemplifies a case in which the differential value in the percentage of muscle is +0.8%, and the differential value in the percentage of body fat is -2.0%. In the example shown in Fig. 6B, the percentage of variation is equal to or more than a%. Accordingly, the background portion of determination display part 5B is illuminated in blue, and further, only the leg part of the human-shape icon is displayed in the blinking manner because the percentage of muscle in the leg part has remarkably increased.

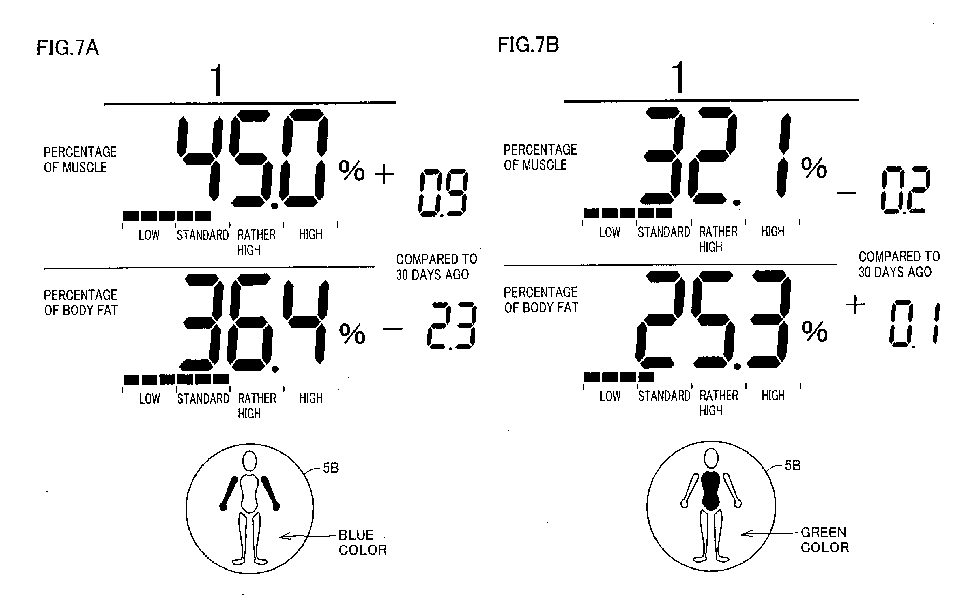

Fig. 7A shows an example of the display on display section 5 in the case in which the arm mode is selected from the respective body part modes, and a result of comparing the measurement result with the data of 30 days ago is displayed. In the display example shown in Fig. 7A, the differential value in the percentage of muscle of an arm part is +0.9% and the differential value in the percentage of body fat of the arm part is -2.3%. In the shown example, the percentage of variation is equal to or more than +a%. Accordingly, the background portion of determination display part 5B is illuminated in blue, and only a relevant arm part of the human-shape icon is illuminated.

Next, Fig. 7B shows an example of the display on display section 5 in which the trunk mode is selected from the respective body part modes and a result of comparing the measurement result with the data measured 30 days ago is displayed. In the display example shown in Fig. 7B, the differential value in the percentage of muscle of the trunk is -0.2% and the differential value in the percentage of body fat of the trunk is +0.1%. It is assumed that, in the shown example, a result obtained when the percentage of variation calculated by means of the percentages of muscle and body fat in the trunk exceeds -a% and falls less than a% is displayed. Accordingly, the background portion of determination display part 5B is illuminated in green, and only a trunk block of the human-shape icon is illuminated.

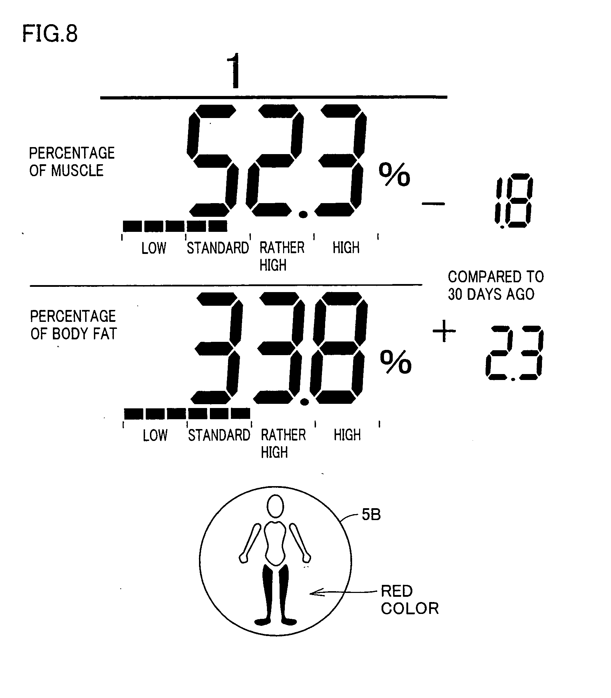

Fig. 8 shows an example of the display on display section 5 in which the leg mode is selected from the respective body part modes and a result of comparing the measurement result with the data measured 30 days ago is displayed. In the example shown in Fig. 8, the differential value in the percentage of muscle in the legs is -1.8%, and the differential value in the percentage of body fat in the legs is +2.3%. It is assumed that, in the shown example, a result obtained when the percentage of variation calculated based on the percentages of muscle and body fat in the legs is equal to or less than -a% is displayed. Accordingly, the background portion of determination display part 5B is illuminated in red, and only the leg block of the human-shape icon is illuminated.

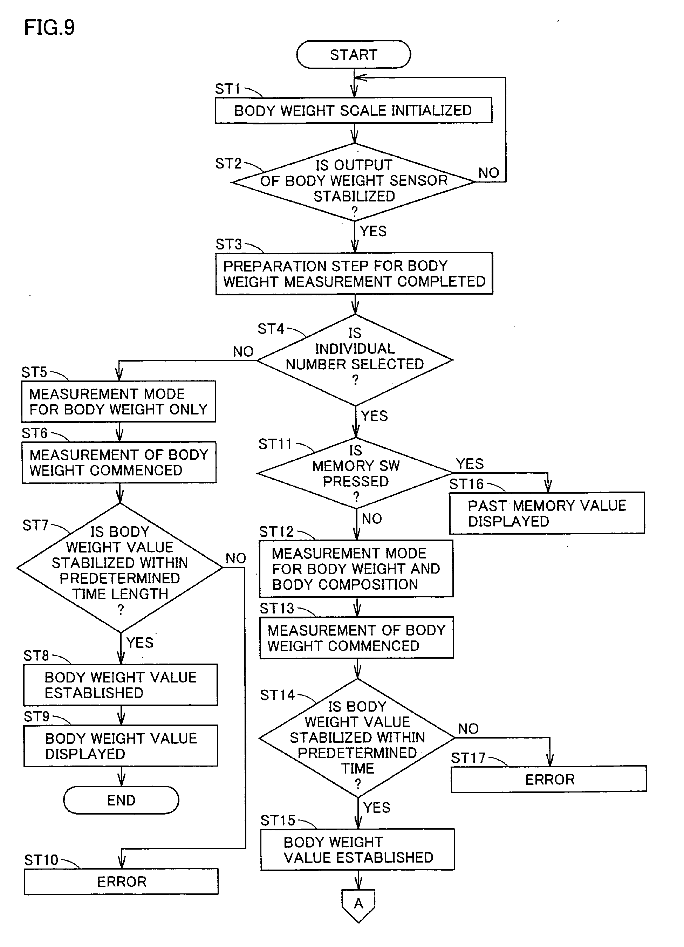

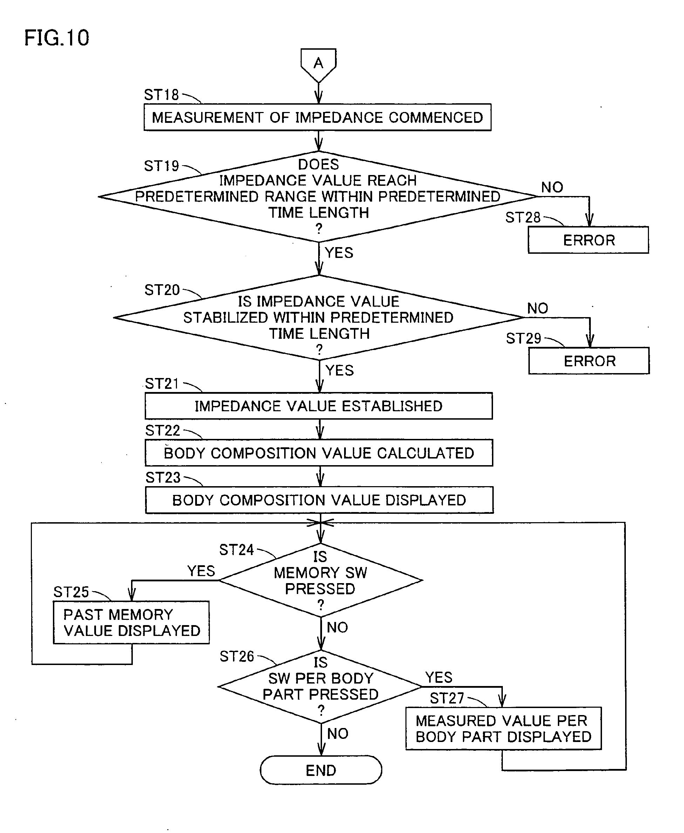

Next, an operation of the measurement process in the body composition measuring device according to the present embodiment is described referring to flowcharts shown in Figs. 9 and 10.

When a power supply is turned on, the process is commenced in body composition measuring device 1, and CPU 25 initializes body weight scale 2 in step ST1. In other words, an output of body weight scale 2 before the subject of measurement is thereon is zero.

CPU 25, then, determines whether or not an output of a body weight sensor is stabilized in step ST2. The body weight sensor is a sensor for detecting the body weight in body weight scale 2. CPU 25 returns the process to step ST1 to repeat the initialization when determining that the output of the body weight sensor is unstable. On the contrary, CPU 25 allows the process to proceed to step ST3 to take on subsequent step upon the determination that the output of the body weight sensor is stabilized.

In step ST3, CPU 25 carries out a setting process to complete a preparation step for measuring the body weight in body weight scale 2, and further allows the process to proceed to step ST4.

In step ST4, CPU 25 determines whether or not a manipulation for selecting an individual number has been executed. When none of keys 151, 152 and 153 of manipulating section 6 is pressed, CPU 25 allows the process to proceed to step ST5. When any of keys 151, 152 and 153 is pressed, CPU 25 allows the process to proceed to step ST11.

In step ST5, CPU 25 instructs the body composition measuring device 1 to enter the measurement mode for the body weight only. Next, in step ST6, CPU 25 commences the weight measurement by body weight scale 2 when the subject of measurement stands on body weight scale 2. Then, CPU 25 allows the process to proceed to step ST7. In step ST7, CPU 25 determines whether or not a measured value of the body weight is stabilized within a predetermined time length, and allows the process to proceed to step ST8 when a variation value of the measured body weight value stays within a range of predetermined values. In contrast, when the variation value of the measured body weight value still stays beyond the range of the predetermined values after the predetermined time length, CPU 25 allows the process to proceed to step ST10 to execute an error process. In step ST8, CPU 25 establishes the measured body weight value. For example, CPU 25 selects an intermediate value in the measured body weight values within a predetermined time length as the established measured value of the body weight, and allows the process to proceed to step ST9. In step ST9, CPU 25 instructs numeral value display part 5A to display the established measured value of the body weight thereon, and thereafter, terminates the process when the power supply is turned off.

In step ST11, CPU 25 determines whether or not a memory SW (memory changeover key 19) is pressed, and allows the process to proceed to step ST16 when it is determined that memory changeover key 19 is pressed. When it is determined that memory changeover key 19 is not pressed, on the contrary, CPU 25 allows the process to proceed to step ST12. In step ST16, CPU 25 reads out a past memory value from external memory 26 and displays it on numeral value display part 5A. When memory changeover key 19 is manipulated once, the data of seven days ago is read out and displayed. Then, whenever memory changeover key 19 is pressed, the data of 30 days ago, the data of 90 days ago, and back to the data of seven days ago again are sequentially read and displayed.

In step ST12, CPU 25 instructs body composition measuring device 1 to enter the measurement mode for the body weight and body composition. In step ST13, CPU 25 commences the measurement of the body weight, and allows the process to proceed to step ST14. In step ST14, CPU 25 determines whether or not a measured body weight value is stabilized within a predetermined time length. CPU 25 allows the process to proceed to step ST15 when it is determined that a variation value of the measured body weight value stays within a range of predetermined values within a predetermined time length. In contrast, when the variation value of the measured body weight value still stays beyond the range of the predetermined values after the predetermined time length, CPU 25 allows the process to proceed to step ST 17 to execute an error process. In step ST15, CPU 25 establishes the measured body weight value. CPU 25, in the same manner as in step ST8, selects an intermediate value in the measured body weight values within a predetermined time length as the established measured value of the body weight, and subsequently allows the process to proceed to step ST18.

In step ST18, CPU 25 commences the measurement of the living-body impedance using living-body impedance measuring section 22, and allows the process to proceed to step ST19. In step ST19, CPU 25 repeats the measurement in real time, while determining whether or not an impedance value detected within a predetermined time length stays within a predetermined range. CPU 25, when the impedance value stays within the predetermined range, allows the process to proceed to step ST20. At that time, it is assumed that the subject of measurement stands on the leg electrodes (right-foot electrodes 8R and 9R and left-foot electrodes 8L and 9L) and holds the right/left-hand electrodes (right-hand electrodes 13R and 14R and left-hand electrodes 13L and 14L). CPU 25 allows the process to proceed to step ST28 to execute an error process when the impedance value still stays beyond the predetermined range after the predetermined time length.

In step ST20, CPU 25 determines whether or not a measured impedance value is stabilized within a predetermined time length. CPU 25 allows the process to proceed to step ST21 when a variation value of the impedance value stays within a range of predetermine values within the predetermined time length. When the variation value of the impedance value still stays beyond the range of the predetermined values after the predetermined time length, CPU 25 allows the process to proceed to step ST29 to execute an error process.

In step ST21, CPU 25 establishes the impedance value. For example, an intermediate value in the impedance values measured within a predetermined time length is used as the established impedance value. Next, CPU 25 allows the process to proceed to step ST22. In step ST22, CPU 25 calculates the body composition such as the amount of body fat, percentage of body fat, amount of lean body mass, percentage of lean body mass, area of visceral fat, amount of visceral fat and percentage of muscle based on the measured body weight value obtained in step ST15, impedance value obtained in step ST21 and personal data previously stored. The foregoing body composition values are stored in external memory 26 as current data with a date of the measurement appended thereto (see Fig. 4). Then, CPU 25 allows the process to proceed to step ST23, wherein CPU 25 instructs numeral value display part 5A to display the obtained body composition values thereon.

The body weight and amount of basal metabolism are first displayed on numeral value display part 5A. Then, whenever setting/display changeover key 18 is pressed, the percentage of muscle in the entire body/percentage of body fat in the entire body, BMI (Body Mass Index)/level of visceral fat, and physical age are sequentially displayed. CPU 25 then allows the process to proceed to step ST24.

In step ST24, CPU 25 determines whether or not memory changeover key 19 is pressed. CPU 25 allows the process to proceed to step ST25 when memory changeover key 19 is pressed. On the other hand, CPU 25 allows the process to proceed to step ST26 when memory changeover key 19 is not pressed. In step ST25, CPU 25 reads out past memory values from external memory 26 and displays them on numeral value display part 5A. If the percentage of muscle in the entire body/percentage of body fat in the entire body are displayed before memory changeover key 19 is pressed, the data measured seven days ago showing the percentage of muscle in the entire body/percentage of body fat in the entire body and the differential value between the current measured data and the data measured seven days ago are displayed when memory changeover key 19 is pressed. After that, whenever memory changeover key 19 is pressed, the measured data of 30 days ago, 90 days ago and seven days go are sequentially read, and the percentages of muscle/body fat in the entire body at each of the foregoing time points and the differential values between the current measured data and the respective measured data from the foregoing time points are displayed. Further, results of determining an increase/decrease detected in the current measured data relative to the respective measured data at the time points are displayed on determination display part 5B (see Figs. 5B to 8).

In step ST26, CPU 25 determines whether or not any body part key is pressed. When any of trunk key 202, leg key 203 and arm key 204 is pressed, CPU 25 allows the process to proceed to step ST27. When the power supply is turned off with none of memory changeover key 19 and the body part keys (trunk key 202, leg key 203 and arm key 204) being pressed, CPU 25 terminates the process.

In step ST27, if memory changeover key 19 has not been pressed yet, the body composition value of the trunk is displayed on numeral value display part 5A. For example, if the percentage of muscle in the entire body/percentage of body fat in the entire body are displayed in step ST23, the percentage of muscle in the trunk/percentage of body fat in the trunk are displayed on numeral value display part 5A in step ST27. When, for example, arm key 204 is pressed, the percentage of muscle in the arm/percentage of body fat in the arm are displayed on numeral value display part 5A.

(Because memory changeover key 19 is pressed) When trunk key 202 is pressed with the percentage of muscle in the entire body/percentage of body fat in the entire body measured seven days ago being displayed on numeral value display part 5A, the percentage of muscle in the trunk/percentage of body fat in the trunk measured seven days ago and the differential value between the current measured data and the measured data of seven days ago showing the percentage of muscle in the trunk/percentage of body fat in the trunk at that time are displayed on numeral value display part 5A in step ST27. At the same time, a result of determining an increase/decrease detected in the current measured data of the trunk relative to the data of the trunk measured seven days ago is displayed on determination display part 5B.

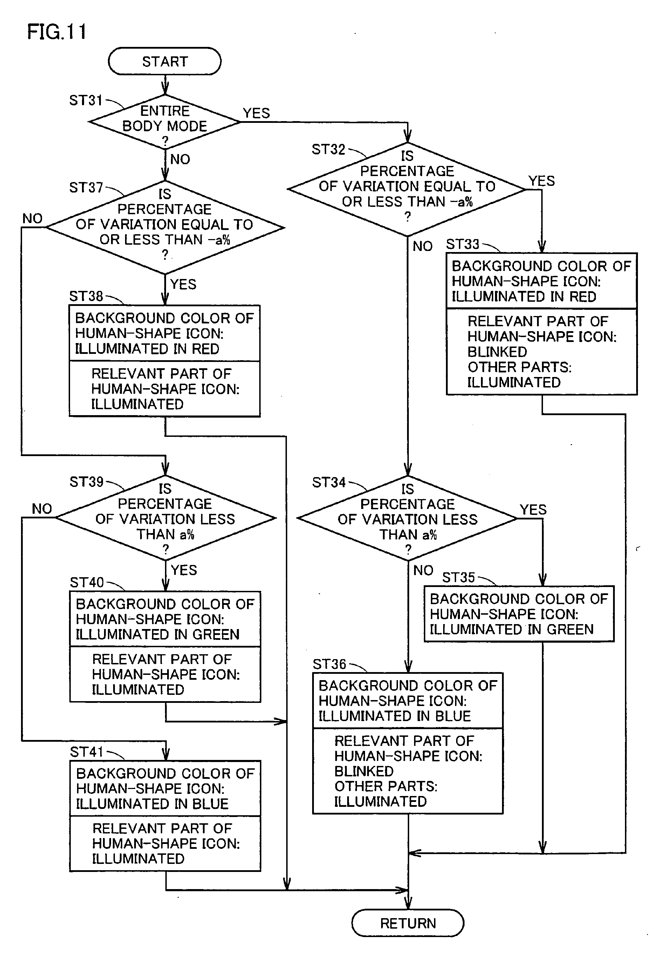

Referring to a flowchart shown in Fig. 11, a process executed for displaying the result of determining the increase/decrease detected in the current measured data relative to the past data is described. In the routine for displaying the determination result, first, CPU 25 determines whether or not the measurement mode is the entire body mode in step ST31. When it is determined that the measurement mode is the entire body mode, CPU 25 allows the process to proceed to step ST32. When the measurement mode is not the entire body mode (when the measurement mode is any of the body part modes), CPU 25 allows the process to proceed to step ST37.

In step ST32, CPU 25 determines whether or not the percentage of variation calculated by means of the previous (past) measured value and the current measured value is equal to or less than -a%. When the percentage of variation is equal to or less than -a%, CPU 25 allows the process to proceed to step ST33. When the percentage of variation exceeds -a%, CPU 25 allows the process to proceed to step ST34.

In step ST33, CPU 25 illuminates the background portion of determination display part 5B in red and checks the respective body parts to thereby blink the block of the human-shape icon corresponding to the body part in which the percentage of variation is equal to or less than -a%. The blocks corresponding to any other body parts are ceaselessly illuminated.

In step ST34, CPU 25 determines whether or not the percentage of variation in the current measured value falls less than a%. When it falls less than a% with a small percentage of variation, CPU 25 allows the process to proceed step ST35. When the percentage of variation does not fall less than a% meaning that it is equal to or more than a%, CPU 25 allows the process to proceed to step ST36.

In step ST35, CPU 25 illuminates the background portion of determination display part 5B in green and does not illuminate the human-shape icon. In step ST36, CPU 25 illuminates the background portion of determination display part 5B in blue and checks the respective body parts to thereby blink the block of the human-shape icon corresponding to the body part in which the percentage of variation is equal to or more than a%. The blocks corresponding to the other body parts are ceaselessly illuminated.

Next, in step ST37, CPU 25 determines whether or not the percentage of variation in the selected body part is equal to or less than -a%. When it is determined that the percentage of variation is equal to or less than -a%, CPU 25 allows the process to proceed to step ST38. When it is determined that the percentage of variation exceeds -a%, CPU 25 allows the process to proceed to step ST39. In step ST38, CPU 25 illuminates the background portion of determination display part 5B in red and further illuminates the block of the human-shape icon corresponding to the selected body part.

In step ST39, CPU 25 determines whether or not the percentage of variation is less than a%. In the case of less than a%, CPU 25 allows the process to proceed to step ST40. In the case of not less than a%, that is, in the case in which the percentage of variation equals to or more than +a%, CPU 25 allows the process to proceed to step ST41.

In step ST40, CPU 25 illuminates the background portion of determination display part 5B in green and further illuminates the block of the human-shape icon corresponding to the selected body part. In step ST41, CPU 25 illuminates the background portion of determination display part 5B in blue, and further, ceaselessly illuminates the block of the human-shape icon corresponding to the selected body part.

In the foregoing embodiment, the measured data stored in the external memory includes the amount/percentage of body fat, percentage of muscle, basal metabolism and the like. As an alternative constitution, only the impedances of the respective body parts may be stored in the storing step, and the amount/percentage of body fat, amount/percentage of muscle and the like may be respectively calculated and used when the stored impedances are called up as the data of seven days ago, 30 days ago and 90 days ago.

Further, the shape of the image displayed on determination display part 5B has the shape of the human body in the foregoing embodiment, however, the image may have a body shape of an animal, robot and the like.

In the foregoing embodiment, the background color of the human-shape icon is changed in response to the comparison result of the measured data. However, the informing method, instead of changing the background color, may employ a different manner using, for example, sound. The body display controller (CPU 25) for controlling the display modes of the human-shape icon displayed on determination display part 5B displays the human-shape icon in different manners, which are, the whole or part thereof is blinked and displayed, the whole or part thereof is ceaselessly illuminated and displayed, and the whole or part thereof is turned off and displayed.

Although the present invention has been described and illustrated in detail, it is clearly understood that the same is by way of illustration and example only and is not to be taken by way of limitation, the spirit and scope of the present invention being limited only by the terms of the appended claims.

**Claims**

1. A body composition measuring device (1) which measures information of a living body such as a percentage of body fat and a percentage of muscle, comprising:

   a measuring section (8R, 8L, 9R, 9L, 13R, 13L, 14R, 14L) which measures said living-body information in an entire body or respective body parts;
   a memory (26) which stores measured data of said living-body information;
   a measured data comparing section (25) which compares the measured data of said living-body information stored in said memory with latest measured data of said living-body information;
   a measured data display section (5A) which displays the measured data of said living body information;
   a body display section (5B) which displays information including an image representing a shape of a human body including respective body parts such as arm and leg; and
   a body display controller (25) which controls display modes of said body display section in accordance with a result of the comparison of the measured data by said measured data comparing section.

2. The body composition measuring device according to claim 1, wherein
   said body display controller controls a background color of the image displayed on said body display section to thereby control the display modes of said body display section.

3. The body composition measuring device according to claim 1, wherein said body display controller controls the display modes of said body display section in accordance with the comparison result of said measured data comparing section when said measured data display section displays the measured data of the entire body.

4. The body composition measuring device according to claim 3, wherein
   said body display controller blinks a part of the image displayed on said body display section corresponding to said entire body or said body part.

5. The body composition measuring device according to claim 3, wherein
   said measured data displayed on said measured data display section is measured data of a plurality of information of the living body.

6. The body composition measuring device according to claim 3, wherein
   said measured data displayed on said measured data display section is a calculation result using the measured data of the plurality of living body information.

7. The body composition measuring device according to claim 6, wherein said plurality of living body information is the percentage of muscle and the percentage of body fat, and
   said calculation result using said plurality of living body information represents a degree of variation detected in the percentage of muscle and the percentage of body fat relative to past measured values.

8. The body composition measuring device according to claim 1, wherein said body display controller controls the display modes of said body display section in accordance with contents displayed on said measured data display section.

9. The body composition measuring device according to claim 8, wherein said body display controller blinks a part of the image displayed on said body display section corresponding to said entire body or said body part.

10. The body composition measuring device according to claim 8, wherein
    said measured data displayed on said measured data display section is measured data of a plurality of information of the living body.

**11.** The body composition measuring device according to claim 8, wherein said measured data displayed on said measured data display section is a calculation result using the plurality of living body information.

**12.** The body composition measuring device according to claim 11, wherein
said plurality of living body information is the percentage of muscle and the percentage of body fat, and
said calculation result using said plurality of living body information represents a degree of variation detected in the percentage of muscle and the percentage of body fat relative to past measured values.

FIG.1

FIG.2

FIG.3

RIGHT-HAND ELECTRODE

LEFT-HAND ELECTRODE

13R(14R)

13L(14L)

BODY WEIGHT SCALE

2

8R(9R)

8L(9L)

RIGHT-FOOT ELECTRODE

LEFT-FOOT ELECTRODE

3

LIVING-BODY IMPEDANCE MEASURING SECTION — 22

SIGNAL CHANGEOVER CIRCUIT — 23

24 — A/D CONVERTING SECTION

26

EXTERNAL MEMORY

25 — CPU

5

6

DISPLAY SECTION

MANIPULATING SECTION

FIG.4

| INDIVIDUAL No. | | AGE | | GENDER | | BODY HEIGHT | | |
|---|---|---|---|---|---|---|---|---|
| WAIST | | | | | | | | |
| MEASURING DATE | BODY WEIGHT | PERCENTAGE OF BODY FAT | AMOUNT OF BODY FAT | PERCENTAGE OF MUSCLE | AMOUNT OF MUSCLE | AMOUNT OF BASAL METABOLISM | AREA OF VISCERAL FAT | AMOUNT OF VISCERAL FAT | MEASURED BODY PART |
| | | | | | | | | | |
| | | | | | | | | | |

14

FIG.5A

PERCENTAGE OF MUSCLE

%

LOW STANDARD RATHER HIGH HIGH

PERCENTAGE OF BODY FAT

%

LOW STANDARD RATHER HIGH HIGH

1

FIG.5B

PERCENTAGE OF MUSCLE

% +

LOW STANDARD RATHER HIGH HIGH

PERCENTAGE OF BODY FAT

% −

LOW STANDARD RATHER HIGH HIGH

COMPARED TO 7 DAYS AGO

1

5B

GREEN COLOR

# FIG.6A

1

PERCENTAGE
OF MUSCLE

**28.8** % − 0.6

LOW  STANDARD  RATHER HIGH  HIGH

COMPARED TO
30 DAYS AGO

PERCENTAGE
OF BODY FAT

**23.0** % + 2.0

LOW  STANDARD  RATHER HIGH  HIGH

— 5B

— RED
COLOR

# FIG.6B

1

PERCENTAGE
OF MUSCLE

**28.8** % + 0.8

LOW  STANDARD  RATHER HIGH  HIGH

COMPARED TO
90 DAYS AGO

PERCENTAGE
OF BODY FAT

**23.0** % − 2.0

LOW  STANDARD  RATHER HIGH  HIGH

— 5B

— BLUE
COLOR

EP 1 576 923 A1

FIG.7A

PERCENTAGE OF MUSCLE

% +

COMPARED TO 30 DAYS AGO

LOW STANDARD RATHER HIGH HIGH

PERCENTAGE OF BODY FAT

% −

LOW STANDARD RATHER HIGH HIGH

5B

BLUE COLOR

FIG.7B

PERCENTAGE OF MUSCLE

% −

COMPARED TO 30 DAYS AGO

LOW STANDARD RATHER HIGH HIGH

PERCENTAGE OF BODY FAT

% +

LOW STANDARD RATHER HIGH HIGH

5B

GREEN COLOR

## FIG.8

PERCENTAGE
OF MUSCLE

**52.3** %

LOW | STANDARD | RATHER HIGH | HIGH

− **18**

COMPARED TO
30 DAYS AGO

PERCENTAGE
OF BODY FAT

**33.8** %

LOW | STANDARD | RATHER HIGH | HIGH

+ **23**

5B

RED COLOR

EP 1 576 923 A1

FIG.9

Flowchart:

START

ST1 — BODY WEIGHT SCALE INITIALIZED

ST2 — IS OUTPUT OF BODY WEIGHT SENSOR STABILIZED ? — NO / YES

ST3 — PREPARATION STEP FOR BODY WEIGHT MEASUREMENT COMPLETED

ST4 — IS INDIVIDUAL NUMBER SELECTED ? — NO / YES

NO branch:

ST5 — MEASUREMENT MODE FOR BODY WEIGHT ONLY

ST6 — MEASUREMENT OF BODY WEIGHT COMMENCED

ST7 — IS BODY WEIGHT VALUE STABILIZED WITHIN PREDETERMINED TIME LENGTH ? — NO / YES

ST8 — BODY WEIGHT VALUE ESTABLISHED

ST9 — BODY WEIGHT VALUE DISPLAYED

END

ST10 — ERROR

YES branch:

ST11 — IS MEMORY SW PRESSED ? — YES / NO

ST16 — PAST MEMORY VALUE DISPLAYED

ST12 — MEASUREMENT MODE FOR BODY WEIGHT AND BODY COMPOSITION

ST13 — MEASUREMENT OF BODY WEIGHT COMMENCED

ST14 — IS BODY WEIGHT VALUE STABILIZED WITHIN PREDETERMINED TIME ? — NO / YES

ST17 — ERROR

ST15 — BODY WEIGHT VALUE ESTABLISHED

A

19

## FIG.10

A

ST18 — MEASUREMENT OF IMPEDANCE COMMENCED

ST19 — DOES IMPEDANCE VALUE REACH PREDETERMINED RANGE WITHIN PREDETERMINED TIME LENGTH ?

NO

ST28 — ERROR

YES

ST20 — IS IMPEDANCE VALUE STABILIZED WITHIN PREDETERMINED TIME LENGTH ?

NO

ST29 — ERROR

YES

ST21 — IMPEDANCE VALUE ESTABLISHED

ST22 — BODY COMPOSITION VALUE CALCULATED

ST23 — BODY COMPOSITION VALUE DISPLAYED

ST24 — IS MEMORY SW PRESSED ?

YES

ST25 — PAST MEMORY VALUE DISPLAYED

NO

ST26 — IS SW PER BODY PART PRESSED ?

YES

ST27 — MEASURED VALUE PER BODY PART DISPLAYED

NO

END

## FIG.11

```
                    ┌─────────────┐
                    │    START    │
                    └──────┬──────┘
                           │
       ST31 ─────◇ ENTIRE BODY MODE ? ◇──── YES ──────────────┐
                           │                                   │
                          NO                      ST32 ─◇ IS PERCENTAGE OF
                           │                       VARIATION EQUAL TO
                           │                       OR LESS THAN -a% ? ◇── YES ─┐
       ST37 ─◇ IS PERCENTAGE OF                         │                      │
         VARIATION EQUAL TO                            NO          ST33 ┌──────────────────────┐
  NO ──  OR LESS THAN -a% ? ◇                           │              │ BACKGROUND COLOR OF   │
         │                                              │              │ HUMAN-SHAPE ICON:     │
        YES                                             │              │ ILLUMINATED IN RED    │
         │                                              │              ├──────────────────────┤
  ST38 ┌──────────────────────┐                         │              │ RELEVANT PART OF      │
        │ BACKGROUND COLOR OF   │                        │              │ HUMAN-SHAPE ICON:     │
        │ HUMAN-SHAPE ICON:     │                        │              │ BLINKED               │
        │ ILLUMINATED IN RED    │                        │              │ OTHER PARTS:          │
        ├──────────────────────┤                         │              │ ILLUMINATED           │
        │ RELEVANT PART OF      │                        │              └──────────────────────┘
        │ HUMAN-SHAPE ICON:     │                        │
        │ ILLUMINATED           │                        │
        └──────────────────────┘                         │
         │                                               │
  ST39 ─◇ IS PERCENTAGE OF                    ST34 ─◇ IS PERCENTAGE OF
  NO ──  VARIATION LESS THAN a% ? ◇            VARIATION LESS THAN a% ? ◇── YES ─┐
         │                                           │                          │
        YES                                         NO           ST35 ┌──────────────────────┐
         │                                           │                │ BACKGROUND COLOR OF   │
  ST40 ┌──────────────────────┐                      │                │ HUMAN-SHAPE ICON:     │
        │ BACKGROUND COLOR OF   │                     │                │ ILLUMINATED IN GREEN  │
        │ HUMAN-SHAPE ICON:     │          ST36 ┌──────────────────────┐
        │ ILLUMINATED IN GREEN  │                │ BACKGROUND COLOR OF   │
        ├──────────────────────┤                 │ HUMAN-SHAPE ICON:     │
        │ RELEVANT PART OF      │                │ ILLUMINATED IN BLUE   │
        │ HUMAN-SHAPE ICON:     │                ├──────────────────────┤
        │ ILLUMINATED           │                │ RELEVANT PART OF      │
        └──────────────────────┘                 │ HUMAN-SHAPE ICON:     │
         │                                        │ BLINKED               │
  ST41 ┌──────────────────────┐                   │ OTHER PARTS:          │
        │ BACKGROUND COLOR OF   │                  │ ILLUMINATED           │
        │ HUMAN-SHAPE ICON:     │                  └──────────────────────┘
        │ ILLUMINATED IN BLUE   │
        ├──────────────────────┤              ┌─────────────┐
        │ RELEVANT PART OF      │              │   RETURN    │
        │ HUMAN-SHAPE ICON:     │              └─────────────┘
        │ ILLUMINATED           │
        └──────────────────────┘
```

21

**European Patent Office**

## EUROPEAN SEARCH REPORT

Application Number

EP 05 00 5517

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| P,X | EP 1 413 250 A (TANITA CORPORATION) 28 April 2004 (2004-04-28) * paragraph [0093] - paragraph [0094] * * paragraph [0101]; figure 13 * ----- | 1,8,10, 11 | A61B5/053 |
| Y | US 2003/073925 A1 (KOMATSU YOSHICHIKA ET AL) 17 April 2003 (2003-04-17) * paragraph [0034] - paragraph [0035] * * paragraph [0045] - paragraph [0048] * * figures 9,10,12 * ----- | 1-12 | |
| Y | US 2002/022773 A1 (DRINAN DARREL ET AL) 21 February 2002 (2002-02-21) * paragraph [0031] - paragraph [0034] * * paragraph [0075] - paragraph [0083] * ----- | 1-12 | |
| A | US 5 458 117 A (CHAMOUN ET AL) 17 October 1995 (1995-10-17) * column 10, line 46 - column 11, line 4 * ----- | 2,4,9 | |
| A | EP 1 386 581 A (TANITA CORPORATION) 4 February 2004 (2004-02-04) * paragraph [0069] * * paragraph [0082]; figure 22 * ----- | 1 | TECHNICAL FIELDS SEARCHED (Int.Cl.7) A61B |
| A | EP 1 095 613 A (TANITA CORPORATION) 2 May 2001 (2001-05-02) * paragraph [0019] * * paragraph [0055] * * paragraph [0059] * ----- | 1 | |

The present search report has been drawn up for all claims

| Place of search | Date of completion of the search | Examiner |
|---|---|---|
| Berlin | 25 May 2005 | Trachterna, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 1 576 923 A1**

## ANNEX TO THE EUROPEAN SEARCH REPORT
## ON EUROPEAN PATENT APPLICATION NO.

EP 05 00 5517

This annex lists the patent family members relating to the patent documents cited in  the above-mentioned European search report.
The members are as contained in the European Patent Office EDP file on
The European Patent Office is in no way liable for these particulars which are merely  given for the purpose of information.

25-05-2005

| Patent document cited in search report | | Publication date | Patent family member(s) | | Publication date |
|---|---|---|---|---|---|
| EP 1413250 | A | 28-04-2004 | JP | 2004141223 A | 20-05-2004 |
| | | | EP | 1413250 A1 | 28-04-2004 |
| | | | US | 2004082877 A1 | 29-04-2004 |
| US 2003073925 | A1 | 17-04-2003 | DE | 60012283 D1 | 26-08-2004 |
| | | | EP | 1092387 A1 | 18-04-2001 |
| | | | EP | 1466554 A1 | 13-10-2004 |
| | | | EP | 1466555 A1 | 13-10-2004 |
| | | | JP | 2001178696 A | 03-07-2001 |
| | | | JP | 2004243148 A | 02-09-2004 |
| | | | US | 6490481 B1 | 03-12-2002 |
| US 2002022773 | A1 | 21-02-2002 | US | 6354996 B1 | 12-03-2002 |
| | | | AU | 3708299 A | 01-11-1999 |
| | | | WO | 9952425 A2 | 21-10-1999 |
| | | | EP | 1071366 A2 | 31-01-2001 |
| US 5458117 | A | 17-10-1995 | US | 5320109 A | 14-06-1994 |
| | | | AT | 240077 T | 15-05-2003 |
| | | | CA | 2191594 A1 | 14-12-1995 |
| | | | DE | 69530768 D1 | 18-06-2003 |
| | | | DE | 69530768 T2 | 18-03-2004 |
| | | | EP | 0764001 A1 | 26-03-1997 |
| | | | ES | 2199994 T3 | 01-03-2004 |
| | | | JP | 10501163 T | 03-02-1998 |
| | | | WO | 9533404 A1 | 14-12-1995 |
| | | | AT | 178198 T | 15-04-1999 |
| | | | CA | 2122032 A1 | 29-04-1993 |
| | | | CA | 2434275 A1 | 29-04-1993 |
| | | | DE | 69228823 D1 | 06-05-1999 |
| | | | DE | 69228823 T2 | 25-11-1999 |
| | | | EP | 0610365 A1 | 17-08-1994 |
| | | | EP | 0898234 A1 | 24-02-1999 |
| | | | JP | 7500983 T | 02-02-1995 |
| | | | WO | 9307804 A1 | 29-04-1993 |
| EP 1386581 | A | 04-02-2004 | JP | 2003024293 A | 28-01-2003 |
| | | | EP | 1386581 A2 | 04-02-2004 |
| | | | CN | 1398572 A | 26-02-2003 |
| | | | EP | 1279366 A2 | 29-01-2003 |
| | | | US | 2003050570 A1 | 13-03-2003 |
| EP 1095613 | A | 02-05-2001 | JP | 2001190514 A | 17-07-2001 |
| | | | CN | 1293944 A | 09-05-2001 |
| | | | DE | 60011367 D1 | 15-07-2004 |
| | | | EP | 1095613 A1 | 02-05-2001 |

EPO FORM P0459

For more details about this annex : see Official Journal of the European Patent Office, No. 12/82

23

**ANNEX TO THE EUROPEAN SEARCH REPORT
ON EUROPEAN PATENT APPLICATION NO.**                     EP 05 00 5517

This annex lists the patent family members relating to the patent documents cited in the above-mentioned European search report.
The members are as contained in the European Patent Office EDP file on
The European Patent Office is in no way liable for these particulars which are merely given for the purpose of information.

25-05-2005

| Patent document cited in search report | Publication date | Patent family member(s) | Publication date |
|---|---|---|---|
| EP 1095613 A | | TW 471958 B <br> US 6516221 B1 | 11-01-2002 <br> 04-02-2003 |

EPO FORM P0459

For more details about this annex : see Official Journal of the European Patent Office, No. 12/82